Europäisches Patentamt

European Patent Office

Office européen des brevets

(19)

(11) Veröffentlichungsnummer: **0 122 585**
**B1**

(12) **EUROPÄISCHE PATENTSCHRIFT**

(45) Veröffentlichungstag der Patentschrift:
08.02.89

(51) Int. Cl.⁴: **C 07 D 501/46, A 61 K 31/545**

(21) Anmeldenummer: **84103977.9**

(22) Anmeldetag: **10.04.84**

(54) Neue Kristallmodifikation von Ceftazidim.

(30) Priorität: **16.04.83 DE 3313818**

(43) Veröffentlichungstag der Anmeldung:
**24.10.84 Patentblatt 84/43**

(45) Bekanntmachung des Hinweises auf die Patenterteilung:
**08.02.89 Patentblatt 89/6**

(84) Benannte Vertragsstaaten:
**AT BE CH DE FR GB IT LI LU NL SE**

(56) Entgegenhaltungen:
**FR-A- 2 466 467**
**FR-A- 2 466 469**
**GB-A- 2 025 398**

(73) Patentinhaber: **HOECHST AKTIENGESELLSCHAFT,
Postfach 80 03 20, D-6230 Frankfurt am Main 80 (DE)**

(72) Erfinder: **Dürckheimer, Walter, Dr., Im Lerchenfeld 45,
D-6234 Hattersheim am Main (DE)**

**Patentansprüche**

Das (6R,7R)-7-[(Z]-2-(2-Aminothiazol-4-yl)-2-(2-carboxyprop-2-oximino)acetamido]-3-(1-pyridiniummethyl)-ceph-3-em-4-carboxylat (Ceftazidim) ist ein Cephalosporin-Antibiotikum der 3. Generation mit einer ausgeprägten Wirksamkeit gegenüber gram-negativen Erregern, einschliesslich der Pseudomonas-Species (vgl. DE-OS 29 21.316). Das Pentahydrat dieser Verbindung (vgl. DE-OS 30 37 102) ist bisher die einzige kristalline Form, die für die parenterale Anwendung gut geeignet ist. Das aus FR-A-2 466 469 bekannte, kristalline Dihydrochlorid stellt eine stark saure Form dar und ist daher für die parenterale Anhwendung weniger gut geeignet.

Es ist bekannt, dass Cephalosporine in ihrer Haltbarkeit begrenzt sind und sich beim Lagern leicht zersetzen. Ursache dafür ist der sehr hydrolyseempfindliche β-Lactamring, der selbst mit kristallgittergebundenem Wasser noch langsam reagieren kann, wenn das Molekül einer Temperaturbelastung ausgesetzt wird oder bei Raumtemperatur über längere Zeit gelagert wird. Es ist deshalb von grosser Bedeutung, eine möglichst stabile Kristallform für die medizinische Anwendung eines Cephalosporin-Antibiotikums zu verwenden.

Es wurde nun gefunden, dass Ceftazidim in eine neue kristalline Modifikation überführt werden kann, die sich gegenüber dem Pentahydrat durch erhöhte thermische Stabilität auszeichnet und für eine parenterale Zubereitung besonders gut geeignet ist.

Gegenstand der Erfindung sind daher ein wasserfreies, kristallisiertes Ceftazidim, seine pharmazeutischen Zubereitungen, sowie ein Verfahren zu seiner Herstellung, das dadurch gekennzeichnet ist, dass man ein kristallisiertes Ceftazidim-Hydrat, vorzugsweise das Pentahydrat, bis zur Gewichtskonstanz dehydratisiert. Der Begriff «wasserfrei» schliesst einen geringen Wassergehalt nicht aus, solange sich dadurch die angegebenen physikalischen Eigenschaften nicht ändern.

Die erfindungsgemässe Kristallmodifikation besitzt ein charakteristisches Debye-Scherrer-Diagramm (Tabelle 1), das das Vorliegen einer neuen Kristallform mit neuen physikalischen und chemischen Eigenschaften belegt.

Als Ausgangsmaterial kommt jedes kristalline Ceftazidim-Hydrat, beispielsweise eines mit 1,5 Mol Wasser (vgl. EP-A-0 122 584), vorzugsweise das Pentahydrat (vgl. DE-OS 30 37 102) in Betracht.

Als dehydratisierende Mittel sind die im Labor dafür üblichen Substanzen verwendbar, wie z.B. Phosphorpentoxid, geglühtes Natriumsulfat, konzentrierte Schwefelsäure oder Calciumchlorid, vorzugsweise Phosphorpentoxid.

Der Dehydratisierungsvorgang kann in den hierfür üblichen technischen Apparaturen, wie z.B. Exsiccatoren, Trockenpistolen oder Trockenschränken durchgeführt werden wobei auch die Anwendung eines Schutzgases, wie beispielsweise Stickstoff, anstelle von Luft möglich ist.

Der Dehydratisierungsvorgang kann unter Normaldruck oder unter vermindertem Druck, beispielsweise bei Wasserstrahlvakuum durchgeführt werden. Bei Arbeiten im Vakuum wird der Vorgang beschleunigt.

Die Dehydratisierung wird im allgemeinen bei Raumtemperatur vorgenommen, kann jedoch auch ohne weiteres bei niedrigeren (beispielsweise 10°C) oder erhöhten (beispielsweise 40°C) Temperaturen ausgeführt werden.

Die Dehydratisierungszeit hängt insbesondere von der Teilchengrösse des Ausgangsmaterials, der Schichthöhe in der angewandten Apparatur und den Dehydratisierungsbedingungen, wie z.B. Temperatur und Druck ab. Die Trocknungszeit kann so beispielswiese zwischen mehreren Stunden und Tagen liegen.

Die verbesserte Stabilität der neuen Kristallmodifikation ergibt sich bei Lagerung der Proben unter Temperaturbelastung im Vergleich zu Ceftazidim-Pentahydrat. Beispielsweise werden je 0,5 g Substanz in einer braunen Glasampulle eingeschmolzen, eine Woche bei 80°C gelagert und anschliessend Gehaltsbestimmungen beispielsweise mittels Hochdrucksflüssigkeitschromatographie (HPLC) durchgeführt. Wie aus Tabelle 2 hervorgeht, wird Ceftazidim-Pentahydrat innerhalb einer Woche nahezu völlig zersetzt, während die erfindungsgemässe Kristallmodifikation noch eine unerwartet hohe Stabilität zeigt und deshalb für die medizinische Anwendung besonders geeignet ist.

Die erfindungsgemässe Verbindung ist ein wertvolles Antibiotikum, das zur Bekämpfung gram-positiver und vor allem gram-negativer Infektionen geeignet ist (vgl. DE-OS 29 21 316).

Gegenstand der Erfindung sind daher auch pharmazeutische Zubereitungen, die die erfindungsgemässe Verbindung enthalten, wie z.B. Lösungen, Suspensionen oder Emulsionen in öligen oder wässrigen Trägern.

Die erfindungsgemässe Verbindung kann als solche oder zusammen mit therapeutisch üblicherweise eingesetzten Hilfsstoffen, wie z.B. Formulierungs-, Lösungs-, Suspendier- und/oder Dispergiermitteln kombiniert werden. Der Wirkstoff kann in einer Zubereitung beispielsweise auch vor seiner Verwendung in Pulverform zur Auflösung in beispielsweise sterilem, pyrogenfreiem Wasser vorliegen. Zum Herstellen von wässrigen Lösungen wird der Wirkstoff zweckmässigerweise durch Zusatz eines basischen Hilfsstoffes, wie z.B. Natriumcarbonat, Natriumbicarbonat, Kaliumbicarbonat, Kaliumcarbonat, Lithiumcarbonat, Calciumcarbonat, Magnesiumcarbonat, Trihydroxymethyl-methylamin, Ethylendiamin, Lysin, Arginin, Glucosamin, N-Methyl-glucosamin, Trihydroxymethyl-ethylamin, Diethanolamin, Diethylamin, Piperazin oder Procain gelöst.

Die Herstellung der Zubereitungen erfolgt in an sich bekannter Weise, z.B. durch Mischen, Einrühren, Lösen usw. mit/in den pharmazeutischen Hilfsstoffen.

Die Dosierung kann in einer Einheitsdosis beispielsweise zwischen etwa 50 und 1.500 mg Wirkstoff liegen, während eine Tagesdosis etwa 0,5 bis 6, vorzugsweise 1 bis 3 g betragen kann.

Beispiel:

20 g Ceftazidim-Pentahydrat werden in einem Exsiccator über Phosphorpentoxid (ca. 500 g) bei Raumtemperatur (20–25%) getrocknet und der Wasserverlust durch tägliche Wägungen des Materials bestimmt. Nach 12 Tagen (Abnahme 2,85 g) bleibt die Probe gewichtskonstant. Man erhält so 17,15 g farbloses, kristallines Ceftazidim in wasserfreier Form.

Der Trocknungsprozess wird durch höhere Temperatur (bis 50 °C) und Vakuum beschleunigt. Das NMR-Spektrum von wasserfreiem Ceftazidim in $CF_3CO_2D$ entspricht bezüglich der C–H Protonenverschiebung vollständig dem des Pentahydrats. Die typischen Absorptionen des Debye-Scherrer-Diagramms, zeigt Tabelle 1.

$C_{22}H_{22}N_6O_7S_2$ (546,6)

|  | C | H | N | S |
|---|---|---|---|---|
| berechnet: | 48,34 | 4,06 | 15,38 | 11,73%; |
| gefunden: | 47,8 | 4,2 | 15,3 | 11,7%. |

Tabelle 1
Charakteristische Kristallbeugungswinkel der wasserfreien, kristallinen Form von Ceftazidim

| Beugungswinkel °2 (Cµ-Kα) | d [A°] | rel. Int. [%] |
|---|---|---|
| 6,75 | 13,1 | 20 |
| 9,0 | 9,82 | 100 |
| 9,15 | 9,65 | 50 |
| 10,94 | 8,08 | 15 |
| 11,50 | 7,69 | 35 |
| 11,85 | 7,47 | 10 |
| 12,65 | 7,0 | 60 |
| 13,05 | 6,77 | 20 |
| 14,30 | 6,19 | 15 |
| 16,40 | 5,40 | 20 |
| 16,95 | 5,23 | 30 |
| 17,45 | 5,08 | 10 |
| 18,80 | 4,72 | 20 |
| 19,50 | 4,55 | 60 |
| 20,45 | 4,34 | 40 |
| 21,0 | 4,23 | 40 |
| 21,95 | 4,05 | 45 |
| 24,05 | 3,70 | 10 |
| 24,65 | 3,61 | 10 |
| 25,30 | 3,52 | 20 |
| 27,05 | 3,29 | 15 |
| 28,45 | 3,14 | 10 |
| 30,90 | 2,89 | 10 |

Tabelle 2
Thermische Stabilität von Ceftazidim-Pentahydrat (A) und wasserfreiem, kristallinem Ceftazidim (B) bei 80° C

| Ceftazidim-Kristall-modifikation | Gehalt der Proben nach HPLC | | |
|---|---|---|---|
|  | 0 Tage | 8 Tage | 15 Tage |
| A | 100% | 0% | 0% |
| B | 100% | 84,2% | 77,5% |

**Patentansprüche für die Vertragsstaaten BE, CH, DE, FR, GB, IT, LI, LU, NL, SE**

1. Wasserfreies, kristallisiertes (6R,7R)-7-[(Z)-2-(2-Aminothiazol-4-yl)-2-(2-carboxyprop-2-oximino)-acetamido]-3-(1-pyridiniummethyl)-ceph-3-em-4-carboxylat, dessen Kristallform im wesentlichen die folgenden Werte eines Debye-Scherrer-Diagramms aufweist:

Tabelle 1
Charakteristische Kristallbeugungswinkel der wasserfreien, kristallinen Form von Ceftazidim

| Beugungswinkel °2 (Cµ-Kα) | d [A°] | rel. Int. [%] |
|---|---|---|
| 6,75 | 13,1 | 20 |
| 9,0 | 9,82 | 100 |
| 9,15 | 9,65 | 50 |
| 10,94 | 8,08 | 15 |
| 11,50 | 7,69 | 35 |
| 11,85 | 7,47 | 10 |
| 12,65 | 7,0 | 60 |
| 13,05 | 6,77 | 20 |
| 14,30 | 6,19 | 15 |
| 16,40 | 5,40 | 20 |
| 16,95 | 5,23 | 30 |
| 17,45 | 5,08 | 10 |
| 18,80 | 4,72 | 20 |
| 19,50 | 4,55 | 60 |
| 20,45 | 4,34 | 40 |
| 21,0 | 4,23 | 40 |
| 21,95 | 4,05 | 45 |
| 24,05 | 3,70 | 10 |
| 24,65 | 3,61 | 10 |
| 25,30 | 3,52 | 20 |
| 27,05 | 3,29 | 15 |
| 28,45 | 3,14 | 10 |
| 30,90 | 2,89 | 10 |

2. Verfahren zur Herstellung des wasserfreien, kristallisierten (6R,7R)-7-[(Z)-2-(2-Aminothiazol-4-yl)-2-(2-carboxyprop-2-oximino)acetamido]-3-(1-pyridiniummethyl)-ceph-3-em-4-carboxylats, dadurch gekennzeichnet, dass man ein kristallines Hydrat dieser Verbindung bis zur Gewichtskonstanz dehydratisiert.

3. Verfahren gemäss Anspruch 2, dadurch gekennzeichnet, dass man als kristallines Hydrat das Pentahydrat einsetzt.

4. Gegen bakterielle Infektionen wirksame pharmazeutische Zubereitungen, gekennzeichnet durch einen Gehalt an kristallisiertem, wasserfreiem (6R,7R)-7-[(Z)-2-(2-Aminothiazol-4-yl)-2-(2-carboxyprop-2-oximino)-acetamido]-3-(1-pyridiniummethyl)-ceph-3-em-4-carboxylat.

5. Verfahren zur Herstellung von gegen bakterielle Infektionen wirksamen pharmazeutischen Präparaten, dadurch gekennzeichnet, dass das wasserfreie, kristallisierte

(6R,7R)-7-[(Z)-2-(2-Aminothiazol-4-yl)-2-(2-carboxyprop-2-oximino)acetamido]-3-(1-pyridiniummethyl)-ceph-3-em-4-carboxylat

gegebenenfalls mit pharmazeutisch üblichen Hilfsstoffen in eine pharmazeutisch geeignete Verabreichungsform gebracht wird.

6. Wasserfreies, kristallines (6R,7R)-7-[(Z)-2-(2-Aminothiazol-4-yl)-2-(2-carboxyprop-2-oximino)acetamido]-3-(1-pyridiniummethyl)-ceph-3-em-4-carboxylat zur Bekämpfung bakterieller Infektionen.

**Patentansprüche für den Vertragsstaat AT**

1. Verfahren zur Herstellung eines wasserfreien, kristallisierten (6R,7R)-7-[(Z)-2-(2-Aminothiazol-4-yl)-2-(2-carboxyprop-2-oximino)acetamido]-3-(1-pyridiniummethyl)-ceph-3-em-4-carboxylats, dessen Kristallform im wesentlichen die folgenden Werte eines Debye-Scherrer-Diagramms aufweist:

Tabelle 1
Charakteristische Kristallbeugungswinkel der wasserfreien, kristallinen Form von Ceftazidim

| Beugungswinkel °2 (Cμ-Kα) | d [A°] | rel. Int. [%] |
|---|---|---|
| 6,75 | 13,1 | 20 |
| 9,0 | 9,82 | 100 |
| 9,15 | 9,65 | 50 |
| 10,94 | 8,08 | 15 |
| 11,50 | 7,69 | 35 |
| 11,85 | 7,47 | 10 |
| 12,65 | 7,0 | 60 |
| 13,05 | 6,77 | 20 |
| 14,30 | 6,19 | 15 |
| 16,40 | 5,40 | 20 |
| 16,95 | 5,23 | 30 |
| 17,45 | 5,08 | 10 |
| 18,80 | 4,72 | 20 |
| 19,50 | 4,55 | 60 |
| 20,45 | 4,34 | 40 |
| 21,0 | 4,23 | 40 |
| 21,95 | 4,05 | 45 |
| 24,05 | 3,70 | 10 |
| 24,65 | 3,61 | 10 |
| 25,30 | 3,52 | 20 |
| 27,05 | 3,29 | 15 |
| 28,45 | 3,14 | 10 |
| 30,90 | 2,89 | 10 |

dadurch gekennzeichnet, dass man ein kristallines Hydrat dieser Verbindung bis zur Gewichtskonstanz dehydratisiert.

2. Verfahren gemäss Anspruch 1, dadurch gekennzeichnet, dass man als kristallines Hydrat das Pentahydrat einsetzt.

**Claims for the contracting states BE, CH, DE, FR, GB, IT, LI, LU, NL, SE**

1. Anhydrous crystalline (6R,7R)-7-[(Z)-2-(2-aminothiazol-4-yl)-2-(2-carboxyprop-2-oximino)acetamido]-3-(1-pyridiniummethyl)-3-cephem-4-carboxylate whose crystalline form essentially exhibits the following values of a Debye-Scherrer diagram:

| Diffraction angle °2 (Cμ-Kα) | d [A°] | rel. int. [%] |
|---|---|---|
| 6.75 | 13.1 | 20 |
| 9.0 | 9.82 | 100 |
| 9.15 | 9.65 | 50 |
| 10.94 | 8.08 | 15 |
| 11.50 | 7.69 | 35 |
| 11.85 | 7.47 | 10 |
| 12.65 | 7.0 | 60 |
| 13.05 | 6.77 | 20 |
| 14.30 | 6.19 | 15 |
| 16.40 | 5.40 | 20 |
| 16.95 | 5.23 | 30 |
| 17.45 | 5.08 | 10 |
| 18.80 | 4.72 | 20 |
| 19.50 | 4.55 | 60 |
| 20.45 | 4.34 | 40 |
| 21.0 | 4.23 | 40 |
| 21.95 | 4.05 | 45 |
| 24.05 | 3.70 | 10 |
| 24.65 | 3.61 | 10 |
| 25.30 | 3.52 | 20 |
| 27.05 | 3.29 | 15 |
| 28.45 | 3.14 | 10 |
| 30.90 | 2.89 | 10 |

2. A process for the preparation of anhydrous crystalline (6R,7R)-7-[(Z)-2-(2-aminothiazol-4-yl)-2-(2-carboxyprop-2-oximino)acetamido]-3-(1-pyridiniummethyl)-3-cephem-4-carboxylate, which comprises dehydrating a crystalline hydrate of this compound to constant weight.

3. The process as claimed in claim 2, wherein the pentahydrate is employed as the crystalline hydrate.

4. Pharmaceutical formulations active against bacterial infections containing crystalline anhydrous (6R,7R)-7-[(Z)-2-(2-aminothiazol-4-yl)-2-(2-carboxyprop-2-oximino)-acetamido]-3-(1-pyridiniummethyl)-3-cephem-4-carboxylate.

5. A process for the preparation of pharmaceutical products active against bacterial infections which comprises converting the anhydrous crystalline (6R,7R)-7-[(Z)-2-(2-aminothiazol-4-yl)-2-(2-carboxyprop-2-oximino)-acetamido]-3-(1-pyridiniummethyl)-3-cephem-4-carboxylate, optionally with customarily used pharmaceutical auxiliaries, into a pharmaceutical form suitable for administration.

6. An anhydrous crystalline
(6R,7R)-7-[(Z)-2-(2-aminothiazol-4-yl)-2-
(2-carboxyprop-2-oximino)-acetamido]-3-(1-
pyridiniummethyl)-3-cephem-4-carboxylate
for controlling bacterial infections.

**Claims for the contracting state AT**

1. A process for the preparation of an anhydrous crystalline
(6R,7R)-7-[(Z)-2-(2-aminothiazol-4-yl)-2-(2-
carboxyprop-2-oximino)acetamido]-3-(1-pyri-
diniummethyl)-3-cephem-4-carboxylate
whose crystalline form essentially exhibits the following values of a Debye-Scherrer diagram, which comprises dehydrating a crystalline hydrate of this compound to constant weight.

| Diffraction angle °2 (Cµ-Kα) | d [A°] | rel. int. [%] |
|---|---|---|
| 6.75 | 13.1 | 20 |
| 9.0 | 9.82 | 100 |
| 9.15 | 9.65 | 50 |
| 10.94 | 8.08 | 15 |
| 11.50 | 7.69 | 35 |
| 11.85 | 7.47 | 10 |
| 12.65 | 7.0 | 60 |
| 13.05 | 6.77 | 20 |
| 14.30 | 6.19 | 15 |
| 16.40 | 5.40 | 20 |
| 16.95 | 5.23 | 30 |
| 17.45 | 5.08 | 10 |
| 18.80 | 4.72 | 20 |
| 19.50 | 4.55 | 60 |
| 20.45 | 4.34 | 40 |
| 21.0 | 4.23 | 40 |
| 21.95 | 4.05 | 45 |
| 24.05 | 3.70 | 10 |
| 24.65 | 3.61 | 10 |
| 25.30 | 3.52 | 20 |
| 27.05 | 3.29 | 15 |
| 28.45 | 3.14 | 10 |
| 30.90 | 2.89 | 10 |

2. The process as claimed in claim 1, wherein the pentahydrate is employed as the crystalline hydrate.

**Revendications pour les Etats contractants BE, CH, DE, FR, GB, IT, LT, LU, NL, SE**

1. (6R,7R)-7-[(Z)-2-(2-aminothiazol-4-yl)-
2-(2-carboxyprop-2-oximino)acétamido]-
3-(1-pyridiniumméthyl)-céph-3-ème-4-
carboxylate
anhydre, cristallin, dont la forme cristalline présente essentiellement les valeurs suivantes d'un diagramme Debye-Scherrer:

| Angle de diffraction °2 (Cµ-Kα) | d [A°] | Int, rel, (%) |
|---|---|---|
| 6,75 | 13,1 | 20 |
| 9,0 | 9,82 | 100 |
| 9,15 | 9,65 | 50 |
| 10,94 | 8,08 | 15 |
| 11,50 | 7,69 | 35 |
| 11,85 | 7,47 | 10 |
| 12,65 | 7,0 | 60 |
| 13,05 | 6,77 | 20 |
| 14,30 | 6,19 | 15 |
| 16,40 | 5,40 | 20 |
| 16,95 | 5,23 | 30 |
| 17,45 | 5,08 | 10 |
| 18,80 | 4,72 | 20 |
| 19,50 | 4,55 | 60 |
| 20,45 | 4,34 | 40 |
| 21,0 | 4,23 | 40 |
| 21,95 | 4,05 | 45 |
| 24,05 | 3,70 | 10 |
| 24,65 | 3,61 | 10 |
| 25,30 | 3,52 | 20 |
| 27,05 | 3,29 | 15 |
| 28,45 | 3,14 | 10 |
| 30,90 | 2,89 | 10 |

2. Procédé de préparation du
(6R, 7R)-7-[(Z)-2-(2-aminothiazol-4-yl)-2-
(2-carboxyprop-2-oximino)acétamido]-3-(1-
pyridiniumméthyl)-céph-3-ème-4-carboxylate
anhydre, cristallisé, caractérisé en ce qu'on déshydrate un hydrate cristallin de ce composé jusqu'à un poids constant.

3. Procédé suivant la revendication 2, caractérisé en ce qu'on utilise comme hydrate cristallin le pentahydrate.

4. Préparations pharmaceutiques active contre les infections bactériennes, caractérisées en ce qu'elles contiennent du
(6R, 7R)-7-[(Z)-2-(2-aminothiazol-4-yl)-2-(2-car-
boxyprop-2-oximino)acétamido]-3-(1-pyri-
diniumméthyl)-céph-3-ème-4-carboxylate
cristallisé, anhydre.

5. Procédé de fabrication de préparations pharmaceutiques actives contre les infections bactériennes, caractérisé en ce qu'on amène sous une forme d'administration pharmaceutiquement appropriée le
(6R, 7R)-7-[(Z)-2-(2-aminothiazol-4-yl)-2-(2-
carboxyprop-2-oximino)acétamido]-3-(1-
pyridiniumméthyl)-céph-3-ème-4-carboxylate
anhydre, cristallisé, le cas échéant avec des adjuvants habituels en pharmacie.

6. (6R, 7R)-7-[(Z)-2-(2-aminothiazol-4-yl)-
2-(2-carboxyprop-2-oximino)acétamido]-
3-(1-pyridiniumméthyl)-céph-3-ème-4-car-
boxylate
anhydre, cristallin, pour la lutte contre les infections bactériennes.

**Revendications pour l'Etat contactant AT**

1. Procédé de préparation d'un (6R, 7R)-7-[(Z)-2-(2-aminothiazol-4-yl)-2-(2-carboxyprop-2-oximino)acétamido]-3-(1-pyridiniumméthyl)-céph-3-ème-4-carboxylate anhydre, cristallisé, dont la forme cristalline présente essentiellement les valeurs suivantes d'un diagramme Debye-Scherrer:

| Angle de diffraction °2 (Cμ-Kα) | d [A°] | Int, rel, (%) |
|---|---|---|
| 6,75 | 13,1 | 20 |
| 9,0 | 9,82 | 100 |
| 9,15 | 9,65 | 50 |
| 10,94 | 8,08 | 15 |
| 11,50 | 7,69 | 35 |
| 11,85 | 7,47 | 10 |
| 12,65 | 7,0 | 60 |
| 13,05 | 6,77 | 20 |
| 14,30 | 6,19 | 15 |
| 16,40 | 5,40 | 20 |
| 16,95 | 5,23 | 30 |

| Angle de diffraction °2 (Cμ-Kα) | d [A°] | Int, rel, (%) |
|---|---|---|
| 17,45 | 5,08 | 10 |
| 18,80 | 4,72 | 20 |
| 19,50 | 4,55 | 60 |
| 20,45 | 4,34 | 40 |
| 21,0 | 4,23 | 40 |
| 21,95 | 4,05 | 45 |
| 24,05 | 3,70 | 10 |
| 24,65 | 3,61 | 10 |
| 25,30 | 3,52 | 20 |
| 27,05 | 3,29 | 15 |
| 28,45 | 3,14 | 10 |
| 30,90 | 2,89 | 10 |

caractérisé en ce qu'on déshydrate un hydrate cristallin de ce composé jusqu'à poids constant.

2. Procédé suivant la revendication 1, caractérisé en ce qu'on utilise comme hydrate cristallin le pentahydrate.